# EUROPÄISCHE PATENTSCHRIFT

(11) **EP 0 711 136 B1**
(45) Veröffentlichungstag und Bekanntmachung des Hinweises auf die Patenterteilung: **17.11.1999**
(21) Anmeldenummer: 94924688.8
(22) Anmeldetag: 01.08.1994
(51) Int. Cl.: A61F 5/01

(54) **SELBSTTÄTIG SPERRENDES GELENK, INSBESONDERE ORTHESENGELENK**
SELF-LOCKING JOINT, IN PARTICULAR ORTHOTIC JOINT
ARTICULATION A BLOCAGE AUTOMATIQUE, NOTAMMENT ARTICULATION D'ORTHESE

(30) Priorität: 30.07.1993 DE 4325655
(43) Veröffentlichungstag der Anmeldung: 15.05.1996
(73) Patentinhaber: Spandau, Kai Henning, D-30177 Hannover (DE)
(72) Erfinder: SPANDAU, Kai, Henning, D-30177 Hannover (DE); KOPP, Franz, Otto, D-30171 Hannover (DE); DZIUBIEL, Marian, D-30926 Seelze (DE)
(74) Vertreter: Brümmerstedt, Hans Dietrich, Dipl.-Ing.
(86) Internationale Anmeldenummer: DE9400890
(87) Internationale Veröffentlichungsnummer: WO9503761

(56) Entgegenhaltungen:
- EP-A- 0 369 786
- AT-B- 384 733
- GB-A- 2 260 083
- US-A- 4 723 539
- US-A- 5 107 824

## Beschreibung

Die Erfindung betrifft ein selbsttätig sperrendes Gelenk, insbesondere ein Orthesengelenk, bestehend aus zwei mittels einer Drehachse verbundenen Anschlußstücken. Über diese Anschlußstücke erfolgt die Verbindung des Gelenks mit den jeweiligen Orthesenteilen.

Gelenke an Orthesen haben eine stabilisierende Wirkung. Sie führen und stützen die Bewegung von teilgelähmten Patienten (z. B. Spina Bifida) und solchen, die nicht in der Lage sind, ihre Bewegung selbst kontrolliert durchzuführen. Derartige Gelenke können, wenn sie eine Stützfunktion ausüben sollen, in einen gesperrten Zustand, und wenn Bewegungsfreiheit für den Patienten gewünscht ist, z. B. wenn er sich setzen möchte, in einen entsperrten Zustand gebracht werden.

Nach dem Stand der Technik sind Zwei grundsätzliche Bauformen derartiger Gelenke bekannt. Herkömmliche Gelenke, die der Behandlungsmethode nach Ferrari entsprechen, werden durch ein einfaches Fallschloß gesperrt, welches eine Relativbewegung der kurzen Hebelarme der Anschlußstücke zueinander im Drehachsenbereich des Gelenks verhindert bzw. mit nur geringem Spiel zuläßt. Zum Entsperren des Gelenks ist das Fallschloß nach oben zu schieben, wodurch das Gelenk freigegeben wird. Aufgrund der Körperhaltung des behinderten Patienten in der Orthese wirken auf das gesperrte Gelenk und damit auf das Fallschloß jedoch sehr große Momente, die ein Entsperren des Gelenks durch den Patienten unmöglich machen und auch von Hilfskräften sehr große Kraftanstrengungen verlangen.

Die andere Bauform der die Hüftbewegung stützenden Gelenke betrifft die sogenannten reziproken Gehorthesen. Bei diesen Gelenken wird ein freischwingendes Element hinzugefügt, während eines der anderen Gelenkanschlußstücke mit einer Wippe (oder Bowdenzügen) zur mechanischen Kraftübertragung von einem Hüftgelenk auf das andere verbunden wird. Gesperrt wird dieses Gelenk durch Einrasten eines Stiftes in einen um die Drehachse des Gelenks drehbaren Gelenkausleger. Bei der Entriegelung dieses Stiftes treten ebenfalls die oben geschilderten Probleme auf.

In der EP 0 369 786 A1 ist ein weiteres selbstsperrendes Gelenk für eine Beinschiene beschrieben. Dieses bekannte Gelenk besteht aus zwei über eine Drehachse verbundenen Anschlußstücken, wobei jedes Anschlußstück eine Kurvenkontur endlicher Breite in Form von Reibflächen aufweist, die, wenn sie in Eingriff miteinander sind, Kräfte von einem zum anderen Anschlußstück übertragen sowie eine Schwenkbewegung zwischen den beiden Anschlußstücken verhindern. In allen möglichen Stellungen der Anschlußstücke verlaufen deren Kurvenkonturen parallel zueinander.

Ausgehend von diesem Stand der Technik ist es Aufgabe der vorliegenden Erfindung, ein selbsttätig sperrendes Gelenk, insbesondere Orthesengelenk zur Verfügung zu stellen, das mit einem so geringen Kraftaufwand entsperrbar ist, daß es selbst für den behinderten Patienten möglich ist, das Gelenk zu entsperren.

Erfindungsgemäß wird diese Aufgabe bei einem gattungsgemäßen Gelenk dadurch gelöst, daß die Kurvenkonturen sich unter einem 50 großen Winkel schneiden, daß ein in den beiden Kurvenkonturen geführter Rollkörper, dessen Achse im Schnittpunkt der Mittellinien der Kurvenkonturen liegt, bei Drehbewegung des einen Anschlußstückes gegen das andere längs der Mittellinien der Kurvenkonturen zwängungsfrei bewegt wird, und daß ein durch Selbsthemmung hervorgerufener gesperrter Zustand des Gelenks dann erreicht ist, wenn der Winkel α zwischen den an die Mittellinien in deren Schnittpunkt angelegt gedachten Tangenten nahe gegen Null geht und die Verbindungslinie der Mittelpunkte der Krümmungskreise der beiden Kurvenkonturen in dem bei Sperrstellung vorliegenden Kreuzungs- oder Berührungspunkt nicht durch die Drehachse des Gelenks verläuft.

Die Lage der Kurvenkonturen in den Anschlußstücken ist so gewählt, daß sie sich in dem Bereich, in dem das Gelenk frei beweglich sein soll, unter einem relativ großen Winkel schneiden. Dieser Winkel muß so groß sein, daß der in den beiden Kurvenkonturen geführte Rollkörper sich nicht zwischen den Kurvenkonturen verklemmt, wodurch das Gelenk gesperrt sein würde. Werden die Anschlußstücke um ihre Drehachse gegeneinander verdreht, kommen die Kurvenkonturen in eine Lage, in der der Schnittwinkel von in ihrem Schnittpunkt angelegt gedachten Tangenten nahe gegen Null geht. In dieser Lage verklemmt sich der Rolkörper bei Krafteinwirkung auf die Anschlußstücke zwischen den beiden Kurvenkonturen, d.h. er überträgt lotrecht zu den Mittellinien der Kurvenkonturen die Kräfte von einem Anschlußstück auf das andere. In Richtung der Mittellinien der Kurvenkonturen ist er in dieser Position kraftfrei (Tangentenschnittwinkel = Null) bzw. fast kraftfrei (Tangentenschnittwinkel annähernd Null), so daß eine nur geringe Kraft erforderlich ist, um den Rollkörper in eine Position zu schieben, in der er wieder zwängungsfrei in den Kurvenkonturen geführt wird, d.h. das Gelenk ist mit nur sehr geringem Kraftaufwand entsperrbar. Der Tangentenwinkel , bei dem die Sperrwirkung eintritt, ist unter anderem auch von der Werkstoffpaarung zwischen Rollkörper und Kurvenkonturen sowie von der Schmierung abhängig.

Da sich das Gelenk aufgrund der geringen Auslösekraft in gewissen Situationen, z.B. bei schnell aufeinander folgenden Lastwechseln sehr leicht ungewollt entsperren könnte, ist eine auf den Rollkörper einwirkende Kraft vorgesehen, die bei gegenseitiger Bewegung der Anschlußstücke vom entsperrten in den gesperrten Zustand die erreichte Sperrstellung hält und sichert. Diese Kraft kann beispielsweise durch Schwerkraft, ein Gummiband, eine Feder oder einen Magneten aufgebracht werden. Aufgrund dieser Sicherungskraft wird die für die Entsperrung des Gelenks erforderliche Auslösekraft nur geringfügig erhöht.

Weitere vorteilhafte Ausgestaltungen dieser Lösung ergeben sich aus den abhängigen Ansprüchen 2 bis 4.

Nach einer weiteren erfindungsgemäßen Lösung ist ein gattungsgemäßes Gelenk so ausgeführt, daß ein Drehpunkt vorgesehen ist, der zwei Lenker an ihrem einen Ende gelenkig verbindet, wobei diese Lenker je mit einem Anschlußstück in Drehpunkten gelenkig verbunden sind, die nicht mit der Drehachse des Gelenks zusammenfallen, und einer oder beide Lenker im gesperrten Zustand des Gelenks an einem Anschlag anliegen, der so angeordnet ist, daß sich die Lenker in diesem Zustand annähernd in Parallellage oder etwas darüber hinaus befinden, wobei eine auf einen oder beide Lenker einwirkende Kraft das Erreichen ihrer Sperrposition bewirkt. Die Sperrposition ist hier bei Parallellage oder annähernder Parallellage der beiden vorgesehenen Lenker erreicht. In dieser Position werden auf die Anschlußstücke wirkende Kräfte über die beiden Lenker und den diese verbindenden Drehpunkt ausschließlich oder fast ausschließlich senkrecht zur Bewegungsbahn des Drehpunktes übertragen. Dieser ist dadurch in Richtung seiner durch die Lenker vorgegebenen Bewegungsbahn quasi kraftfrei. Im übrigen entspricht der Wirkungsmechanismus dem der oben beschriebenen Lösung, so daß an dieser Stelle weitere Erläuterungen nicht erforderlich sind.

Ein Anschlag kann auch bei der ersten Lösung vorgesehen werden. Dieser kann durch das Ende einer Kurvenkontur gebildet oder aber unabhängig davon angeordnet sein.

Weitere vorteilhafte Ausgestaltungen dieser Lösung ergeben sich aus den abhängigen Ansprüchen 6 und 7.

In vorteilhafter Weiterverfolgung des Erfindungsgedankens ist vorgesehen, die "Kurvenkonturlösung" mit der "Lenkerlösung" zu kombinieren, in dem eine der beiden Kurvenkonturen durch einen Lenker ersetzt wird, derart, daß dessen eines Ende mit dem entsprechenden Anschlußstück drehgelenkig verbunden ist, und dessen anderes Ende in der in dem anderen Anschlußstück vorhandenen Kurvenkontur mittels eines Roll- oder Gleitkörpers geführt ist.

Obgleich die Erfindung vorstehend und nachstehend ausschließlich auf Gelenke für Orthesen bezogen ist, liegt es auf der Hand, derartige selbsttätig sperrende Gelenke auch anderweitig einzusetzen, so z. B. für Drehklappen oder Klapptische.

Des weiteren ist es auch möglich, durch eine entsprechende Gestaltung der Kurvenkonturen bzw. der Lenkeranbindung an die Anschlußstücke mehrere Sperrstellungen vorzusehen.

Die Erfindung wird nachstehend anhand von Ausführungsbeispielen näher erläutert. In der dazugehörigen Zeichnung Zeigt:
- Fig. 1.1: eine Seitenansicht eines Orthesengelenks mit zwei Schlitzkurven in entsperrter Stellung,
- Fig. 1.2: das Gelenk nach Fig. 1.1 in gesperrter Stellung,
- Fig. 2: einen Schnitt durch einen Rollkörper nach Fig. 1.1 bzw. 1.2,
- Fig. 3: eine Prinzipskizze eines Orthesengelenks mit zwei Lenkern in der Sperrstellung,
- Fig. 4: ein Orthesengelenk in Seitenansicht mit einer Schlitzkurve und einem Lenker in gesperrter Stellung,
- Fig. 4.1: eine Prinzipskizze nach Figur 4,
- Fig. 4.2: eine Prinzipskizze einer andere Variante dieses Ausführungsbeispieles
- Fig. 5: das Orthesengelenk gemäß Fig. 4 in entsperrter Stellung, und
- Fig. 6: eine perspektivische Darstellung eines erfindungsgemäßen Gelenks in der Ausführung mit einer Kurvenkontur und einem Lenker für reziproke Gehorthesen.

In den einzelnen Figuren sind gleiche bzw. gleich wirkende Teile mit gleichen Bezugszeichen versehen.

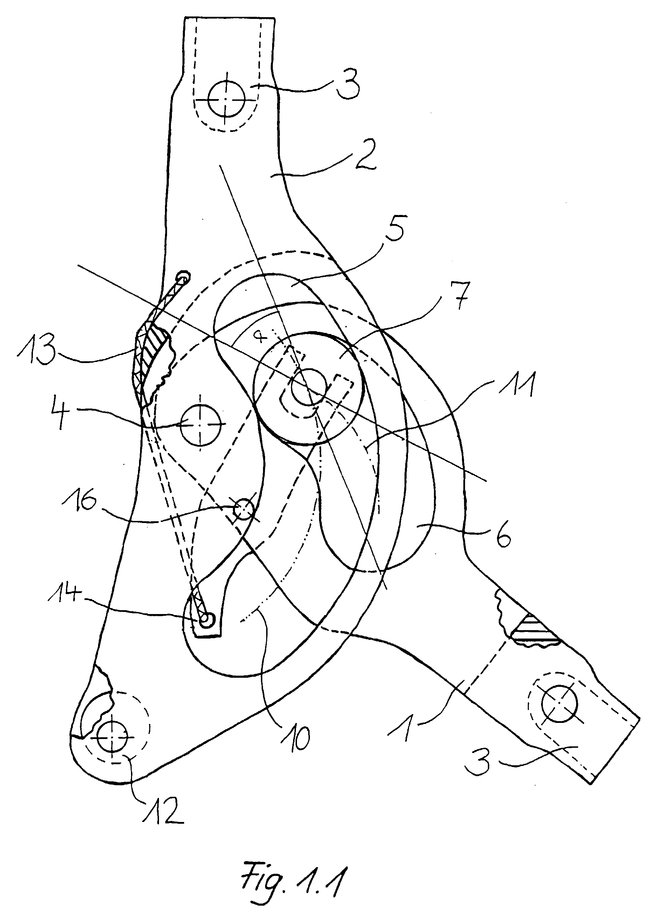

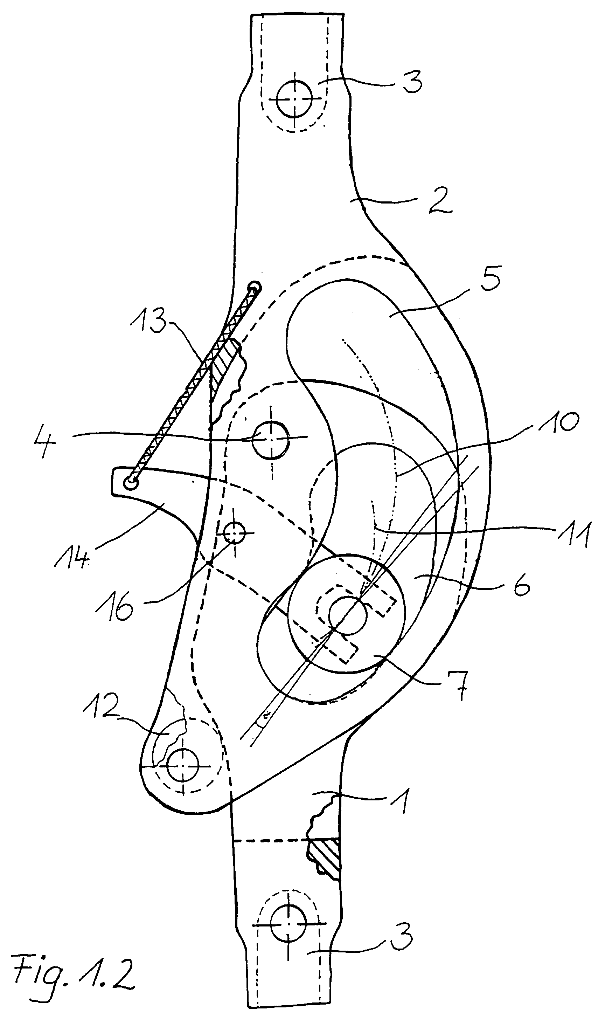

Bei dem in Figur 1 dargestellten Ausführungsbeispiel ist das Gelenk im entsperrten (Fig. 1.1), also frei beweglichen Zustand und im gesperrten Zustand (Fig. 1.2) dargestellt. Die Anschlußstücke 1, 2 weisen Adapter 3 zur Aufnahme und Befestigung von Schienen auf, die ihrerseits am Rücken- bzw. Beinteil der Orthese befestigt werden. Da die Orthese als solche nicht Gegenstand der Erfindung ist, sind diese Teile, auch in den anderen Figuren, nicht dargestellt.

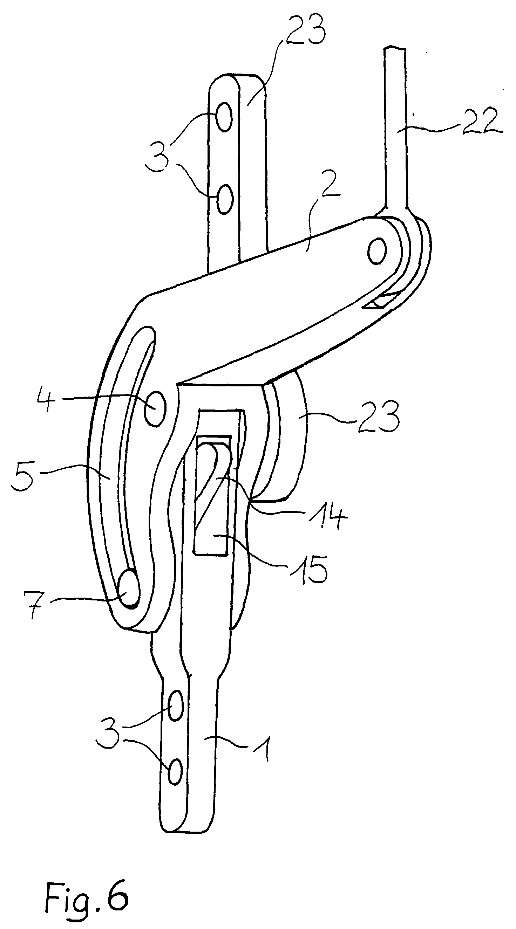

Die Anschlußstücke 1, 2 besitzen eine gemeinsame Drehachse 4, um die sie gegeneinander verschwenkbar sind. Das Anschlußstück 2 ist, was aus der Darstellung nicht ersichtlich ist, gegabelt. In dieser Gabel ist das Anschlußstück 1 angeordnet. Im Prinzip ist diese Konstruktion aus der perspektivischen Darstellung der ein anderes Ausführungsbeispiel betreffenden Figur 6 ersichtlich, auf die zum besseren Verständnis von Figur 1 verwiesen wird.

Die Anschlußstücke 1, 2 besitzen jeweils eine solche Ausdehnung, daß Schlitzkurven 5, 6 vorgesehen werden können, die die Form von Kreisbahnsegmenten haben, und beispielsweise auf einfache Weise mittels eines Fingerfräsers herstellbar sind. Diese Schlitzkurven 5, 6 sind so angebracht, daß sie sich im vorgegebenen Schwenkbereich des Gelenkes kreuzen, wobei die aus der Seitenansicht nach Figur 1 ersichtliche Schlitzkurve 5 in dem hinteren Gabelteil des Anschlußstückes 2 identisch vorhanden ist.

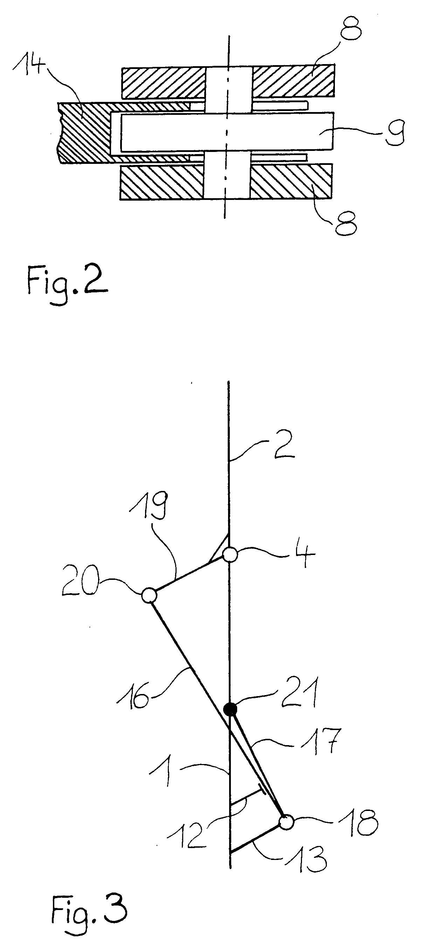

In den Schlitzkurven 5, 6 ist ein Rollkörper 7 geführt. Die Breite der Schlitzkurven 5, 6 und damit der Durchmesser des Rollkörpers 7 ist zur Verringerung der Reibung möglichst groß gewählt. Zur Verringerung der Reibung trägt auch eine Ausführung des Rollkörpers 7 bei, wie er aus der Schnittdarstellung nach Fig. 2 ersichtlich ist. Diese zeigt drei voneinander unabhängig drehbare Rollen, wobei die Rollen 8 in den Schlitzkurven 5 des vorderen bzw. hinteren Gabelteiles des Anschlußstückes 2 laufen und Rolle 9 in der Schlitzkurve 6 des Anschlußstückes 1 läuft.

Aus der Darstellung nach Fig. 1.1 ist erkennbar, daß sich die Schlitzkurven 5 und 6 bzw. ihre Mittellinien 10 und 11 in der entsperrten Stellung des Gelenks mit einem relativ großen Winkel schneiden. Das garantiert eine zwängungsfreie Führung des Rollkörpers 7. Je mehr sich die Lage des Anschlußstückes 1 der Sperrstellung (Fig. 1.2) nähert, desto kleiner wird der Schnittwinkel, um schließlich in der Sperrstellung annähernd Null oder gleich Null zu sein. In dieser Stellung liegt das Anschlußstück 1 an einem zwischen den Gabelteilen des Anschlußstückes 2 angeordneten Ansschlag 12 an. Dieser Anschlag 12 ist als Exzenter ausgeführt, so daß die Endlage des Anschlußstückes 1 und damit die Größe der Auslösekraft einstellbar ist. Anstelle des Anschlages 12 kann auch das Ende der Schlitzkurve 5 als Anschlag genutzt werden.

Die Sperrlage des Anschlußstückes 1 bzw. des Rollkörpers 7 wird durch ein Gummiband 13 gesichert, das über einen Entriegelungshebel 14 auf den Rollkörper 7 wirkt, und diesen auch in der Sperrposition hält. Der Entriegelungshebel 14 ist in einem im Anschlußstück 1 vorgesehenen Schlitz (siehe zur Anschauung Bezugszeichen 15 in Figur 6) angeordnet und um eine im Anschlußstück 1 festgelegte Schwenkachse 16 schwenkbar. Sein eines gabelförmig ausgeführtes Ende (siehe Figur 2) nimmt die Achse des Rollkörpers 7 auf, während sein anderes Ende über das Anschlußstück 2 hinausragt. An diesem Ende ist das Gummiband 13 befestigt, welches zwecks Ausübung eine Zugwirkung entsprechend am Anschlußstück 2 festgelegt ist.

Zum Entsperren des Gelenks ist der Entriegelungshebel 14 an seinem freien Ende nach unten zu drücken, wodurch der Rollkörper 7 aus seiner Klemmlage herausbewegt wird. Das Gelenk ist nun frei beweglich.

Eine besonders einfache Ausführungsform der Erfindung ist in der Prinzipskizze nach Figur 3 dargestellt. Dieses Gelenk weist ebenfalls zwei um eine gemeinsame Drehachse 4 schwenkbare Anschlußstücke 1, 2 auf. Des weiteren sind zwei Lenker 16, 17 vorgesehen, die an ihrem einen Ende über einen Drehpunkt 18 gelenkig miteinander verbunden sind. An ihrem anderen Ende sind die Lenker 16, 17 über einen eine Abwinklung des Lenkers 2 bildenden Ausleger 19 und einen Drehpunkt 20 drehgelenkig mit dem Anschlußstück 2 bzw. über einen Drehpunkt 21 drehgelenkig mit dem Anschlußstück 1 verbunden.

Die Sperrstellung des Gelenks ist erreicht, wenn sich die Lenker 16, 17 in Parallellage oder in annähernder Parallellage befinden. In dieser Position liegt der Lenker 16 an einem Anschlag 12 an, der eine Weiterbewegung des Gelenks über die Totlage hinaus verhindert. Die Sperrstellung wird durch ein zwischen dem Anschlußstück 1 und dem Drehpunkt 18 verspanntes Gummiband 13 gesichert. Zum Entsperren des Gelenkes sind die Lenker 16, 17 aus ihrer Parallellage herauszudrücken, wozu ebenfalls eine geringe Auslösekraft erforderlich ist.

Der Drehpunkt 18 entspricht vom Prinzip her dem Rollkörper 7 (bzw. dessen Achse) des vorherigen Ausführungsbeispiels, wobei in dem einen Fall die Zwangsführung dieses Punktes (Rollkörper 7) durch zwei Schlitzkurven erfolgt und in diesem Fall durch die beiden Lenker 16, 17, deren Endpunkte relativ zu den Anschlußstücken 1 bzw. 2 Kreisbahnen beschreiben, deren Radius jeweils durch die Länge der Lenker 16 bzw. 17 bestimmt ist.

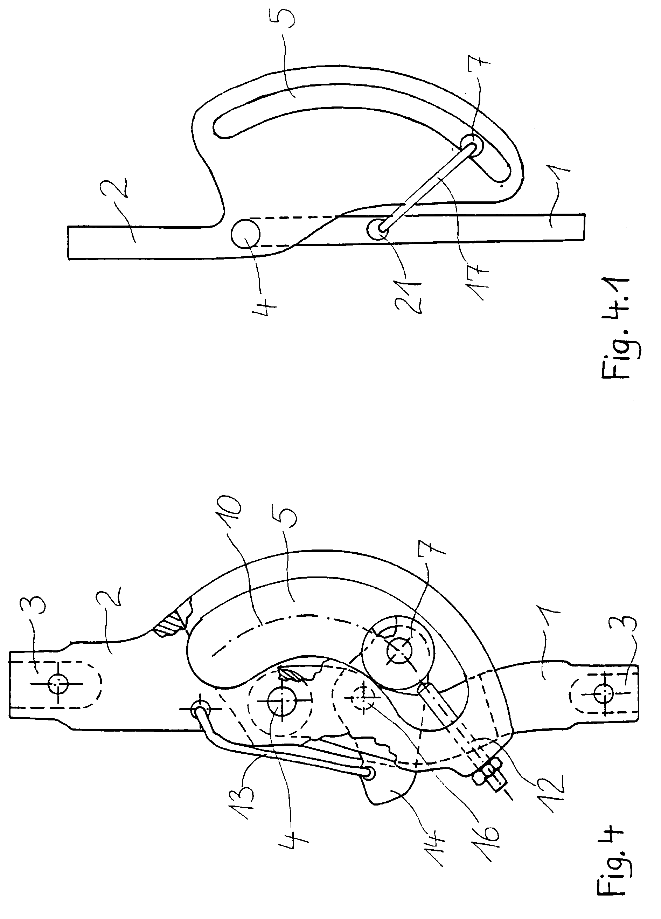

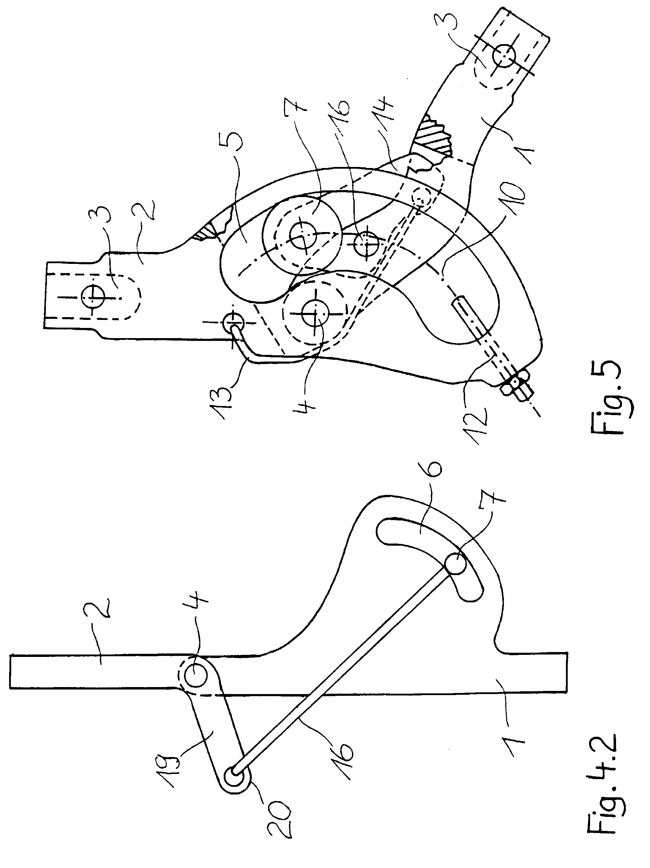

Ein weiteres Ausführungsbeispiel der Erfindung ist in den Figuren 4 und 5 dargestellt. Dieses Ausführungsbeispiel entspricht einer Kombination der Ausführung gemäß den Figuren 1 und 3. Zur Verdeutlichung dieser Kombination ist neben Figur 4 eine dieser Figur entsprechende Prinzipskizze (Figur 4.1) dargestellt.

Aus diesen Darstellungen ist ersichtlich, daß bei diesem Ausführungsbeispiel die Schlitzkurve 6 des Ausführungsbeispiels nach Figur 1 durch den Lenker 17 des Ausführungsbeispieles nach Figur 3 ersetzt worden ist. Natürlich ist es auch möglich, umgekehrt vorzugehen, d.h. die Schlitzkurve 5 durch den Lenker 16 zu ersetzen. Diese Variation ist in Figur 4.2 dargestellt.

Wie auch schon im Ausführungsbeispiel nach Figur 1 ist hier das Anschlußstück 2 zur Aufnahme des Anschlußstückes 1 gegabelt. Die dargestellte Schlitzkurve 5 ist in beiden Gabelteilen identisch vorhanden. Der Rollkörper 7 besteht aus zwei über eine Drehachse verbundenen Rollen, die jeweils in den beiden Schlitzkurven 5 des Anschlußstückes 2 geführt sind. Der Entriegelungshebel 14 bildet gleichzeitig den die Schlitzkurve 6 ersetzenden Lenker, dessen Länge sich aus dem Abstand zwischen der Schwenkachse 16 des Entriegelungshebels 14 und dem Achsmittelpunkt des Rollkörpers 7 ergibt.

Die Wirkungsweise dieser Gelenkausführung ergibt sich in adäquater Weise aus den vorhergehenden Ausführungsbeispielen, so daß es hierzu an dieser Stelle keiner weiteren Erläuterungen bedarf.

Das Ausführungsbeispiel nach Figur 6 zeigt, daß das erfindungsgemäße Gelenk ohne weiteres auch für reziproke Gehorthesen einsetzbar ist. Diese Ausführung entspricht im wesentlichen der Ausführung nach den Figuren 4 und 5. Im Gegensatz dazu ist hier aber das Anschlußstück 2 abgewinkelt. Es wird an seinem abgewinkelten Ende mit der mechanischen Kraftübertragung (Bowdenzüge oder Wippe) verbunden. In Figur 6 ist beispielsweise ein ein zur Wippe führendes, drehbar im Anschlußstück 2 gelagertes Verbindungselement 22 dargestellt. Zusätzlich ist bei diesem Ausführungsbeispiel ein auf der Drehachse 4 des Gelenks frei schwenkbares Verbindungselement 23 zum Rückenteil der Orthese vorgesehen.

## Patentansprüche

1. Selbsttätig sperrendes Gelenk, insbesondere Orthesengelenk, bestehend aus zwei über eine Drehachse (4) verbundenen Anschlußstücken (1, 2), die jeweils mit einer Kurvenkontur (5, 6) endlicher Breite versehen sind, dadurch gekennzeichnet, daß diese Kurvenkonturen (5, 6) sich unter einem so großen Winkel schneiden, daß ein in den beiden Kurvenkonturen (5, 6) geführter Rollkörper (7), dessen Achse im Schnittpunkt der Mittellinien (10, 11) der Kurvenkonturen (5, 6) liegt, bei Drehbewegung des einen Anschlußstückes (1, 2) gegen das andere längs der Mittellinien (10, 11) der Kurvenkonturen (5, 6) zwängungsfrei bewegt wird, und daß ein durch Selbsthemmung hervorgerufener gesperrter Zustand des Gelenks dann erreicht ist, wenn der Winkel α zwischen den an die Mittellinien (10, 11) in deren Schnittpunkt angelegt gedachten Tangenten nahe gegen Null geht und die Verbindungslinie der Mittelpunkte der Krümmungskreise der beiden Kurvenkonturen (5, 6) in dem bei Sperrstellung vorliegenden Kreuzungs- oder Berührungspunkt nicht durch die Drehachse (4) des Gelenks verläuft.

2. Selbsttätig sperrendes Gelenk nach Anspruch 1, dadurch gekennzeichnet, daß bei gegenseitiger Bewegung der Anschlußstücke (1, 2) vom entsperrten in den gesperrten Zustand eine auf den Rollkörper (7) einwirkende Kraft die erreichte Sperrstellung hält und sichert.

3. Selbsttätig sperrendes Gelenk nach Anspruch 1 oder 2, dadurch gekennzeichnet, daß die Kurvenkonturen (5, 6) Abschnitte von Kreisbahnen sind.

4. Selbsttätig sperrendes Gelenk nach einem der vorstehenden Ansprüche, dadurch gekennzeichnet, daß die Kurvenkonturen (5, 6) als Schlitze ausgefürt sind.

5. Selbsttätig sperrendes Gelenk, insbesondere Orthesengelenk, bestehend aus zwei über eine Drehachse (4) verbundenen Anschlußstücken (1, 2), dadurch gekennzeichnet, daß ein Drehpunkt (18) vorgesehen ist, der zwei Lenker (16, 17) an ihrem einen Ende gelenkig verbindet, wobei diese Lenker (16, 17) je mit einem Anschlußstück (1, 2) in Drehpunkten (20, 21) gelenkig verbunden sind, die nicht mit der Drehachse (4) zusammenfallen, und einer der beiden Lenker (16, 17) im gesperrten Zuzstand des Gelenks an einem Anschlag (12) anliegt, der so angeordnet ist, daß sich die Lenker (16, 17) in diesem Zustand annähernd in Parallellage oder etwas darüber hinaus befinden.

6. Selbsttätig sperrendes Gelenk nach Anspruch 5, dadurch gekennzeichnet, daß bei gegenseitiger Bewegung der Lenker (16, 17) vom entsperrten in den gesperrten Zustand eine auf einen oder beide Lenker (16, 17) einwirkende Kraft die erreichte Sperrstellung hält und sichert.

7. Selbsttätig sperrendes Gelenk nach Anspruch 5 oder 6, dadurch gekennzeichnet, daß der Anschlag (12) verstellbar ist.

8. Selbsttätig sperrendes Gelenk nach einem der Ansprüche 1 bis 4 und einem der Ansprüche 5 bis 7, dadurch gekennzeichnet, daß eine der beiden Kurvenkonturen (5, 6) durch einen Lenker (16, 17) ersetzt ist, derart, daß dessen eines Ende mit dem entsprechenden Anschlußstück (1, 2) drehgelenkig verbunden ist, und dessen anderes Ende in der in dem anderen Anschlußstück vorhandenen Kurvenkontur mittels eines Roll- oder Gleitkörpers geführt ist.

## Claims

1. Self-locking joint, in particular orthotic joint, consisting of two connecting pieces (1, 2) joined via a hinge pin (4), each of which connecting pieces is provided with a curved profile (5, 6) of finite width, characterised in that said curved profiles (5, 6) intersect at as large an angle as possible, in that, on rotary movement of the one connecting piece (1, 2) towards the other, a roller element (7), guided in the two curved profiles (5, 6), the axis of which roller element is located at the point of intersection of the mid lines (10, 11) of the curved profiles (5, 6), is moved, free from jamming, along the mid lines (10, 11) of the curved profiles (5, 6), and in that a locked position of the joint produced by self-locking is achieved when the angle α between the theoretical tangent fitted to the mid lines (10, 11) at their point of intersection becomes close to zero and the line joining the mid points of the circles of curvature of the two curved profiles (5, 6) does not pass through the hinge pin (4) of the joint at the crossing point or contact point which exists in the locked position.

2. Self-locking joint according to Claim 1, characterised in that on mutual movement of the connecting pieces (1, 2) from the unlocked into the locked position a force acting on the roller element (7) maintains and secures the locked position achieved.

3. Self-locking joint according to Claim 1 or 2, characterised in that the curved profiles (5, 6) are sections of circular paths.

4. Self-locking joint according to one of the preceding claims, characterised in that the curved profiles (5, 6) are constructed as slits.

5. Self-locking joint, in particular orthotic joint, consisting of two connecting pieces (1, 2) joined via a hinge pin (4), characterised in that a point of rotation (18) is provided which joins two connecting rods (16, 17) in an articulated manner at their one end, said connecting rods (16, 17) each being connected in an articulated manner to one connecting piece (1, 2) at points of rotation (20, 21) which are not coincident with the hinge pin (4) and, when the joint is in the locked position, one of the two connecting rods (16, 17) bearing against a stop (12) that is so arranged that, in this position, the connecting rods (16, 17) are approximately in a parallel position or somewhat beyond this.

6. Self-locking joint according to Claim 5, characterised in that on mutual movement of the connecting rods (16, 17) from the unlocked into the locked position, a force acting on one or both connecting rods (16, 17) maintains and secures the locked position achieved.

7. Self-locking joint according to Claim 5 or 6, characterised in that the stop (12) is adjustable.

8. Self-locking joint according to one of Claims 1 to 4 and one of Claims 5 to 7, characterised in that one of the two curved profiles (5, 6) is replaced by a connecting rod (16, 17) in such a way that one end thereof is joined to the relevant connecting piece (1, 2) so as to produce a rotary articulated joint, and the other end of which is guided by means of a roller or slider element in the curved profile present in the other connecting piece.

## Revendications

1. Articulation à blocage automatique, en particulier articulation d'orthèse, comprenant deux pièces de raccordements (1, 2) qui sont reliées par un axe de rotation (4) et dont les bouts larges ont respectivement des profils courbes (5, 6), caractérisée en ce que ces profils courbes (5, 6) se coupent sous un angle si grand qu'un corps de roulement (7) qui est guidé dans les deux profils courbes et dont l'axe se situe au point d'intersection des lignes médianes (10, 11), se déplace sans contrainte, par rotation d'une pièce de raccordement (1, 2) contre l'autre, le long des lignes médianes (10, 11) des profils courbes (5, 6), et en ce qu'on a alors un état bloqué de l'articulation par blocage automatique, quand l'angle α entre les tangentes imaginaires des lignes médianes (10, 11) à leur intersection tend vers zéro et que la ligne, qui relie les centres des cercles de courbure des deux profils courbes (5, 6), ne passe pas par l'axe de rotation (4) de l'articulation au point de croisement ou de contact en position bloquée.

2. Articulation à blocage automatique suivant la revendication 1, caractérisée en ce que, par mouvement réciproque des pièces de raccordements (1, 2) de l'état débloqué à l'état bloqué, une force agissant sur le corps de roulement (7) tient et assure le blocage atteint.

3. Articulation à blocage automatique suivant la revendication 1 ou 2, caractérisée en ce que les profils courbes (5, 6) sont des portions de glissières circulaires.

4. Articulation à blocage automatique suivant l'une des revendications précédentes, caractérisée en ce que les profils courbes (5, 6) sont réalisées sous forme de fentes.

5. Articulation à blocage automatique, en particulier articulation d'orthèse, comprenant deux pièces de raccordements (1, 2) reliées par un ce de rotation (4), caractérisée en ce qu'un point de rotation (18) est prévu qui relie deux bielles (16, 17) articulées à leurs extrémités, ces bielles (16, 17) étant respectivement reliées et articulées avec un pièce de raccordement (1, 2) en des points de rotation (20, 21), qui ne coïncident pas avec l'axe de rotation (4), et qu'une des deux bielles (16, 17), à l'état bloqué de l'articulation, se situe sur une butée (12) qui est prévue de façon que les bielles (16, 17), dans cet état, se trouvent approximativement en position parallèle ou un peu au-delà.

6. Articulation à blocage automatique suivant la revendication 5, caractérisée en ce que, par mouvement réciproque des bielles (16, 17) de l'état débloqué à l'état bloqué, une force agissant sur une ou les deux bielles (16, 17) tient et assure le blocage atteint.

7. Articulation à blocage automatique suivant la revendication 5 ou 6, caractérisée en ce que la butée (12) est réglable.

8. Articulation à blocage automatique suivant l'une des revendications 1 à 4 et l'une des revendications 5 à 7, caractérisée en ce qu'un des deux profils courbes (5, 6) en remplacé par une bielle (16, 17) de manière qu'une extrémité de celle-ci est reliée articulé en rotation avec la pièce de raccordement correspondante (1, 2) et que son autre extrémité est guidée, dans le profil courbe prévu dans l'autre pièce de raccordement, au moyen d'un corps de roulement ou de glissement.
